# EUROPEAN PATENT APPLICATION

(11) **EP 1 849 499 A2**
(43) Date of publication of application: **31.10.2007**
(21) Application number: 06008601.4
(22) Date of filing: 26.04.2006
(51) Int. Cl.: A61Q 5/08, A61Q 5/10, A61K 8/19, A61K 8/22, A61K 8/42, A61K 8/45

(54) **Amide surfactant thickening systems for hair colouring and bleaching compositons**

(71) Applicant: The Procter and Gamble Company, Cincinnati, Ohio 45202 (US)
(72) Inventor: Bureiko, Andrei Sergeevich, Berkshire SL5 0BB (GB); Hayward, Adam Simon, Durham DH1 5YX (GB); McMeekin, Anthony, Surrey GU19 5QY (GB)
(74) Representative: Kohol, Sonia

(57) **Abstract**

The present invention relates to hair colouring and hair bleaching compositions comprising a source of carbonate ions, at least one oxidizing agent and a specified amide surfactant. The compositions surprisingly provide improved hair colourant and bleaching compositions which deliver improved lift, lightening and colour delivery whilst minimizing damage which are easy to manufacture and have long shelf life stability.

## Description

### FIELD OF THE INVENTION

The present invention relates to amide surfactant based thickening systems for hair colouring and or bleaching compositions.

### BACKGROUND OF THE INVENTION

The permanent alteration of the colour of keratinous fibres, in particular human hair, by the application of hair dyes is well known. In order to provide the consumer with the hair colour and the intensity of colour desired, a very complex chemical process is utilized. Permanent hair dyeing formulations typically comprise oxidative hair dye precursors, which can diffuse into the hair through the cuticle and into the cortex where they can then react with each other and suitable oxidising agents to form the end dye molecules. Due to the larger size of these resultant molecules they are unable to readily diffuse out of the hair during subsequent washing with water and/or detergents; hence delivering a consumer-desired permanency of colour. This reaction typically takes place in an aggressive environment at approximately pH 10 in the presence of an alkalizing agent and in the presence of an oxidizing agent. Moreover, the consumer repeats this process regularly in order to maintain the desired hair colour and shade and the intensity of colour and to ensure continual, even coverage of the hair including coverage of new hair growth.

The manufacturer of such products is also required to work within a large number of constraints. Since these products are being placed in direct contact with the consumers' skin, the potential exists for accidental contact with the eye or for ingestion (for example), which can occur during the dyeing process. Therefore, the formulation must meet rigorous safety requirements and not induce any allergic reactions. In addition to meeting these requirements, the products must also be optically and olfactory pleasing to the consumer. In particular, the products also need to meet certain physical parameters in order to ensure that the product can be easily applied to the hair by the consumer to provide the desired effect, without unintentional staining of the consumers' clothes, skin or other objects.

The manufacturer is also required to provide the hair colouring consumer a large range of different resulting colours. Some consumers may just wish to enhance the natural colour of the hair, whilst others may wish to cover grey or completely alter the hair colour to a different natural appearing hair colour or a 'synthetic' appearing hair colour. Consequently, the manufacturer may provide over twenty different formulations, of varying colours and shades, to address the range of consumer specific needs. These formulations have to be individually formulated and are typically complex formulae containing a mixture of different dye compounds. As a result the manufacture of such product ranges can be costly and complex.

Typically permanent hair dye products will contain a source of alkali such as an ammonia source. This serves the purpose of swelling the hair allowing the entry of the dye precursor molecules into the hair and also improves the lightening effect of the oxidising agent, which is typically hydrogen peroxide. However, ammonia is also volatile and its associated odour is extremely unpleasant to the consumers' of such products, particularly as these hair dye products are used in close proximity to the nasal region. Hence, it would be highly desirable to provide an oxidative hair colouring and/or bleaching composition, that delivers the consumer required lightening level and colour, but which has reduced or climinated the detectable ammonia odour.

In fact another deficiency area in current hair colouring products is the provision of hair colouring products which deliver the required hair lightening effect. Delivering the required level of lightening is particularly important in order to provide the full range of colour shades demanded by the consumer, especially for blonde shades and grey coverage. Such products pose particular difficulties to the manufacturer, as they usually require the use of high levels of oxidising agent and ammonia in order to deliver the required lightening effect. However, in additional to the problems associated with the presence of high levels of ammonia in these products, as discussed herein above, the presence of these high levels of ammonia and/or oxidizing agent also affect the condition of the hair and may in some cases induce mild skin irritation on the scalp. In particular, the hydrophilicity of the hair surface is increased during the colouring process, which alters the sensory perception of the hair and its overall manageability during, and immediately after colouring and during the subsequent wash and styling cycles until the next colourant application. Hence, it would also be highly desirable to provide an oxidative hair colouring and/or bleaching composition which delivers the required lightening and/or colour without unnecessary hair damage.

A number of attempts have been described in the literature to address at least some of the above identified improvement areas. For example the use of carbonate has been described in the following hair colouring art.

EP 435 012 describes hair-dyeing compositions, which require a short dyeing time, create little damage to hair, and no irritating odour after dyeing comprising a carbonate source, a non odour generating alkali hydrogen peroxide and a buffer solution. Similarly EP 1 106 166 describes hair dye compositions comprising ammonia, carbonate (other than ammonia salt), transition metal salt and chelating agent which do not give off an irritating odour, have low skin irritation and can change the hair colour into a lighter tone in a short time. WO01/28508 describes hair colouring formulations comprising oxidising agents and ammonia carbonate or carbamate which deliver improved bleaching and colouring with reduced odour and hair damage without the need for buffering agents, pH modifiers or hair swelling agents. JP01206825 describes a low pungent hair colouring composition comprising ammonia, ammonium salt and carbonate. US2004/0083557 describes hair colouring compositions comprising an oxidative hair dye precursor, a metal cyanate, an alkalizing agent and an oxidizing agent and preferably a metal bicarbonate salt in order to provide good colour lift and low odour.

WO04/014328 describes one step hair colouring compositions comprising peroxide oxidizing agents, specific oxidizing agents and at least one water soluble carbonate releasing salts which more effectively deliver colour wherein the composition is applied for a period of from 2 to 60 minutes. US2004/0098814 describes a method of permanently colouring hair whereby the hair is subjected to a number of consecutive short treatments whereby the treatment comprises a dye intermediate in a shampoo or conditioner base, a water soluble carbonate releasing salt and a water soluble ammonium salt. US2004/0098816 also describes a method for the gradual permanent colouring of hair which includes subjecting the hair to a number of treatments having a set time interval between them, wherein the treatment compositions comprise ammonium carbonate in combination with a chelant.

EP1484047 and EP148447 describe hair colouring compositions comprising a source of carbonate ions and oxidizing agents and a source of radical scavengers to provide improved hair colouring without odour and hair damage.

It has now however been found that the incorporation of hydrogen peroxide and carbonate hair colourant systems, results in difficulties in manufacturing such products. This problem is particularly manifest for hair colouring compositions which have high levels of peroxide and carbonate which are desirable to provide high levels of lift. In order to provide a product which the consumer can easily apply to the hair without dripping onto the skin, eyes, clothes or bathroom surfaces, hair colourant products are designed such that the composition applied on head has a certain required viscosity. This is either achieved by providing the dye composition and the oxidizing composition as so called 'thin-thin-thick' type liquid formulations which are thickened upon mixing. Alternatively, at least one of the components, either the dye composition or the oxidizing composition, preferably the dye composition, is provided as a thickened formulation which thickens the total composition upon mixing, which is a so called 'thick-thin-thick' formulation. Finally, the desired viscosity may also be provided by the use of so called 'thick-thick-thick' formulations wherein both the dye composition and the oxidizing compositions are provided as thickened formulations which upon mixing form a thickened total composition.

The above described viscosity manipulation can typically be achieved by the use of high concentrations of solvent systems. However, such solvent systems are not desirable in terms of skin mildness. Alternatively, thickening systems based upon polymers as described for example in EP1047375 may be used. These systems purport to provide consumer acceptable rheology to the product such that it can be easily applied to the hair whilst importantly not dripping onto the skin or eyes during treatment. However, these materials have also been found not to sufficiently thicken compositions comprising high levels of carbonate resulting in product instability or unsatisfactory viscosity. Moreover, many of these systems do not allow easy mixing of the components by the consumer resulting in inhomogeneous mixtures.

Hence it would be desirable to provide a hair colorant composition which incorporates high levels of carbonate or indeed any other ions without compromising the product stability or ease of manufacture.

Another particularly critical performance area for the consumer is the provision of the desired resultant colour and also the effective coverage of grey hair. Indeed, whilst the amount of grey hair to be coloured varies considerably from consumer to consumer, the resultant overall appearance of the coloured hair demanded by the consumer should be nearly identical for the naturally pigmented hair and the grey hair on head, with the added requirement that the initial coverage is maintained during the post dyeing washing and drying cycle. In order to effectively incorporate the dyes into the compositions, again, typically high levels of solvent are required. However as discussed hereinabove this is not desirable.

Hence, it would be further desirable to provide the consumer with a hair colourant, providing improved lift and lightening and improved colour delivery, uptake and durability and which is easy to manufacture, delivering the required viscosity, without the need for the presence of high solvent levels and which is shelf life stable.

It has now been surprisingly found that oxidative hair colouring compositions comprising an oxidizing agent, a carbonate ion source and an amide surfactant selected from polyoxyethylene amides or polyhydroxy amides and mixtures thereof can be formulated as stable thickened systems which can be utilized in 'thin-thin-thick'systems, 'thin-thick-thick' systems and 'thick-thick-thick' systems. Moreover, the compositions of the present invention are compatible with current dyes and dye precursor systems and result in excellent dye deposition and colour and improved grey coverage. In addition, the compositions exhibit low odour and deliver a high level of lift and lightening equal to the currently utilized ammonia/peroxide systems, whilst reducing the concentration of peroxide and reducing the hair fibre damage.

### SUMMARY OF THE INVENTION

The present invention relates to a hair colouring and or bleaching composition comprising at least 0.25 mole/l of a source of carbonate, carbamate, hydrogencarbonate or peroxymonocarbonate ions and mixtures thereof, at least one oxidizing agent and at least one amide surfactant selected from polyoxyethylene amide or polyhydroxy amides or mixtures thereof.

The present invention also relates to a hair colouring and or bleaching kit comprising
i) an individually packaged first oxidising component comprising at least one oxidizing agent preferably hydrogen peroxide and
ii) an individually packaged second component comprising at least one amide surfactant selected from polyoxyethylene alkanolamide or polyhydroxy alkanolamides or mixtures thereof and a source of carbonate, carbamate, hydrogencarbonate or peroxymonocarbonate ions.

### DETAILED DESCRIPTION OF THE INVENTION

While the specification concludes with claims, which particularly point out and distinctly claim the invention, it is believed the present invention will be better understood from the following description. As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the compositions according to the present invention.

All percentages are by weight of the total composition unless specifically stated otherwise. When more than one composition are used during a treatment, the total weight to be considered is the total weight of all the compositions applied on the hair simultaneously (i.e. the weight found "on head") unless otherwise specified. All ratios are weight ratios unless specifically stated otherwise. All molar concentrations are by weight of the total composition and presented as number of moles of component(s) in one kilogram of the composition, or "mole/kg".

The present invention relates to hair ,colouring and or bleaching and or highlighting compositions.

### Amide Surfactant

The formulation of viscous, strongly associated non-ionic surfactant based, phases in the presence of high level of carbonate ions has been found to pose many difficulties. However, such non-ionic based systems are desirable for their mildness and for their compatibility with cationic conditioning agents. It has now been surprisingly found that the amide surfactants having functionality selected from polyoxyethylene or polyhydroxy form desirable viscous gels when mixed with a source of carbonate ions. Without being bound by theory, this is believed to be due to the ability of the amide group to intermolecularily hydrogen bond (ie. associate) and form strong associated structures or phases.

It has been surprisingly found that the amide surfactants of the present invention can be utilized to provide viscosity for hair colouring and or bleaching systems. The viscosity can be formed by utilizing said surfactant alone as the primary thickener or in combination with other surfactants which are otherwise incompatible with such a high level of carbonate ions. When combined with less salt tolerant surfactants, it is believed that the amide surfactant of this invention prevents the dehydration of the surfactant head groups preventing precipitation and allowing the surfactants to provide viscosity to the system.

The system is particularly beneficial for application of the so called thin-thin type liquid hair colouring or bleaching formulations, whereby upon mixing the two components, the viscosity is increased to the desired level required for application. Furthermore, the systems of the present invention further allow for exceptional ease of mixing ensuring homogeneity for the consumer.

The hair colouring and or bleaching compositions of the present invention comprise an amide surfactant selected from polyoxyethylene amides or polyhydroxy amides. Wherein said amide surfactant is a polyoxyethylene amide according to the formula:
R-(OCH₂CH₂)ₓ-(OCH₂)_{y}-C(O)NH(CH₂CH₂O)₂-H, wherein x is independently selected from 0 to 100, y is 0 or 1, z is independently selected from I to 100, and R is independently selected from alkyl, alkenyl or alkylaryl groups having from 8 to 30 carbon atoms or is a polyhydroxy amide according to the formula:
RC(O)N(R')CH₂-(CHOH)ₙ-H, wherein n is independently selected from 4 to 6, R' is hydrogen or methyl group, R is independently selected from alkyl, alkenyl or alkylaryl groups having from 8 to 30 carbon atoms. Preferred materials are available from Hatco Corporation. A particularly preferred material is laurylmethyl glucamide (HATCO HXL 7658).

The hair colouring and or bleaching compositions typically will comprise from 1% to 60% by weight and more preferably from 5% to 40% of said amide surfactant or mixtures thereof.

### Oxidizing agent

The compositions according to the present invention thus form peroxymonocarbonate ions. These ions are typically formed in *in-situ* from the reaction between a source of hydrogen peroxide and carbonate ion. Consequently, the compositions according to the present invention comprise or are used in combination with a composition that comprises at least one source of an oxidizing agent. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1% to about 15% by weight, preferably from about 1% to about 15% by weight, and most preferably from about 3% to about 12% by most preferably from about 2% to 7% weight of an oxidizing agent.

### Carbonate ion source

According to the present invention the compositions comprise at least about 0.25 mole/l of a source of carbonate ions or carbamate ions or hydrogencarbonate ions or peroxymonocarbonate ions or any mixture thereof. This amount can be achieved for example by addition of at least about 2.40% (volume percent) of ammonium carbonate (molecular weight equals to 96.09 g/mol) to the composition of invention or, for example, by addition of about 1.0% (volume percent) of Ammonium Carbonate and at least about 1.46% (volume percent) of Potassium Hydrogen Carbonate (molecular weight equals 100.12 g/mor). The compositions of the present invention preferably comprises from about 0.4 mole/l to about 2.0 mole/l, more preferably from about 0.5 mole/l to about 1.5 mole/l of the source of said ions.

It should also be understood that when the composition of the invention is used as a hair colouring or bleaching kit comprising an individually packaged oxidizing component and an individually packaged second component such as a bleaching or colouring component, the concentration of the source of the said ions will be increased in the said bleaching or colouring component proportionally to the mixing ratio of components in order to achieve the concentration of at least about 0.25 mole/l upon mixing of the components to provide the composition applied to the hair.

Any source of these ions may be utilized. Suitable sources for use herein include sodium, potassium, lithium, calcium, magnesium, barium, ammonium salts of carbonate, carbamate and hydrogencarbonate ions and mixtures thereof such as sodium carbonate, sodium hydrogen carbonate, potassium carbonate, potassium hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate, carbamate and hydrogencarbonate ions are sodium hydrogen carbonate, potassium hydrogen carbonate, ammonium carbamate and mixtures thereof.

In a particularly preferred embodiment of the present invention, the ammonium ion source and the carbonate ion sources are provided by a single source such as ammonium carbonate, ammonium hydrogen carbonate, ammonium carbamate or mixtures thereof.

According to the present invention the compositions comprise from about 1% to about 35% by weight, preferably from about 5% to about 20% by weight, said source of carbonate, carbamamte, hydrogencarbonate or peroxymonocarbonate ions

### Additional components

The compositions of the present invention may further comprise additional ingredients which include, but are not limited to; alkalising agents, additional surfactants, hair dyeing agents such as oxidative dye precursors, non-oxidative pre-formed dyes, additional thickeners and / or rheology modifiers, opacifiers such as mica, solvents, enzymes, conditioning agents, carriers, antioxidants, stabilizers, chelants, perming actives, perfume, reducing agents, hair swelling agents and/or polymers. Some of these additional components are detailed hereafter.

### Source of alkalizing agent

According to the present invention the composition may optionally comprise at least one source of alkalizing agent, preferably a source of ammonium ions and or ammonia. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium hydrogen carbonate, ammonium carbamate, ammonia and mixtures thereof. The compositions of the present invention may comprise from about 0.1% to about 10% by weight, preferably from about 0.5% to about 5%, most preferably from about 1% to about 3% of an alkalizing agent, preferably ammonium ions. Preferably, if present, the ammonium ions and carbonate ions are present in the composition at a weight ratio of from 3:1 to 1:10, preferably 2:1 to 1:5.

Preferably, the compositions of the present invention have a pH of from about 11 to about 7.5, more preferably from about 9.5 to about 8.4 and most preferably from about 9.4 to about 8.5 and even more preferably about pH 9.0. The pH of the compositions can be determined by using either a Mettler Toledo MP220 or a MP225 pH equipment, fitted with a standard laboratory pH electrode. The equipment is calibrated before each use using standard calibration buffers and using standard calibration procedure.

### Hair dyes

The hair compositions of the present invention are preferably hair colouring compositions which comprise but are not limited to oxidative dyeing compositions. Such compositions comprise oxidative hair dye precursors also known as primary intermediates and couplers that will deliver a variety of hair colors to the hair. These small molecules are activated by the oxidizing agent and react with further molecules to form a larger colored complex in the hair shaft.

The precursors can be used alone or in combination with other precursors, and one or more can be used in combination with one or more couplers. Couplers (also known as color modifiers or secondary intermediates) are generally colorless molecules that can form colors in the presence of activated precursors, and are used with other precursors or couplers to generate specific color effects or to stabilize the color. The choice of precursors and couplers will be determined by the color, shade and intensity of coloration that is desired. The precursors and couplers can be used herein, singly or in combination, to provide dyes having a variety of shades ranging from ash blonde to black.

These compounds are well known in the art, and include aromatic diamines, aminophenols, aromaticdiols and their derivatives (a representative but not exhaustive list of oxidation dye precursor can be found in Sagarin, "Cosmetic Science and Technology", "Interscience, Special Edn. Vol. 2 pages 308 to 310). It is to be understood that the precursors detailed below are only by way of example and are not intended to limit the compositions and processes herein. These are:
1,7-Dihydroxynaphthalene (1,7-NAPHTHALENEDIOL), 1,3-Diaminobenzene (m-PHENYLENEDIAMINE), I-Methyl-2,5-diaminobenzene (TOLUENE-2,5-DIAMINE), 1,4-Diaminobenzene (p-PHENYLENEDIAMINE), 1,3-Dihydroxybenzene (RESORCINOL), 1,3-Dihydroxy-4-chlorobenzene, (4-CHLORORESORCINOL), 1-Hydroxy-2-aminobenzene, (o-AMINOPHENOL), I-Hydroxy-3-aminobenzene (m-AMINOPHENOL), 1-Hydroxy-4-amino-benzene (p-AMINOPHENOL), 1-Hydroxynaphthalene (1-NAPHTHOL), 1,5-Dihydroxynaphthalene (1,5-NAPHTHALENEDIOL), 2,7-dihydroxynaphthalene (2,7-NAPHTHELENEDIOL) 1-Hydroxy-2,4-diaminobenzene (4-DIAMINOPHENOL), 1,4-Dihydroxybenzene (HYDROQUINONE), 1-Hydroxy-4-methylaminobenzene (p-METHYLAMINOPHENOL), 6-Hydroxybenzo-morpholine (HYDROXYBENZOMORPHOLINE), 1-Methyl-2-hydroxy-4-aminobenzene (4-AMINO-2-HYDROXY-TOLUENE), 3,4-Diaminobenzoic acid (3,4-DIAMINOBENZOIC ACID), 1-Methyl-2-hydroxy-4-(2'-hydroxyethyl)aminobenzene (2-METHYL-5-HYDROXYETHYLAMINO-PHENOL), 1,2,4-Trihydroxybenzene (1,2,4-TRIHYDROXYBENZENE), 1-Phenol-3-methylpyrazol-5-on (PHENYLMETHYLPYRAZOLONE), 1-(2'-Hydroxyethyloxy)-2,4-diaminobenzene (2,4-DIAMINOPHENOXY-ETHANOL HCL), 1-Hydroxy-3-arnino-2,4-dichlorobenzene (3-AMINO-2,4-DICHLORO-PHENOL), 1,3-Dihydroxy-2-methylbenzene (2-METHYLRESORCINOL), 1-Amino-4-bis-(2'-hydroxyethyl)aminobenzene (N,N-BIS(2-HYDROXY-ETHYL)-p-PHENYLENE-DIAMINE), 2,4,5,6-Tetraaminopyrimidine (HC Red 16), 1-Hydroxy-3-methyl-4-aminobenzene, (4-AMINO-m-CRESOL), 1-Hydroxy-2-amino-5-methylbenzene (6-AMINO-m-CRESOL), 1,3-Bis-(2,4-Diaminophenoxy)propane (1,3-BIS-(2,4-DIAMINO-PHENOXY)-PROPANE),1 -(2'-Hydroxyethyl)-2,5-diaminobenzene (HYDROXYETHYL-p-PHENYLENE DIAMINE SULPHATE), 1-Methoxy-2-amino-4-(2'-hydroxyethylamino)benzene, (2-AMINO-4-HYDROXYETHYLAMINOANlSOLE) 1-Hydroxy-2-methyl-5-amino-6-chlorobenzene (5-AMINO-6-CHILORO-o-CRESOL), 1-Hydroxy-2-amino-6-methylbenzene (6-AMINO-o-CRESOL), 1-(2'-Hydroxyethyl)-amirio-3,4-methylenedioxybenzene (HYDROXYETHYL-3,4-METHYLENEDIOXY-ANILINE HC1), 2,6-Dihydroxy-3,4-dimethylpyridine (2,6-DIHYDROXY-3,4-DIMETHYLPYRIDINE), 3,5-Diarnino-2,6-dimethoxypyridine (2,6-DIMETHOXY-3,5-PYRIDINEDLAMNE), 5,6-Dihydroxyindole ,(DIHYDROXY-INDOLE), 4-Amino-2-aminomethylphenol (2-AMINOETHYL-p-AMINO-PHENOL HCl), 2,4-Dzamino-5-methylphenetol (2,4-DIAMINO-5-METHYL-PHENETOLE HCl), 2,4-Diamino-5-(2'-hydroxyethyloxy)toluene (2,4-DIAMINO-5-METHYLPHENOXYETHANOL HC1), 5-Amino-4-chloxo-2-methylphenol (5-AMINO-4-CHLORO-o-CRESOL), 4-Amino-l-hydroxy-2-(2'-hydroxyethylaminomethyl)benzene HYDROXYETHYLAMINOMETHYL-p-AMINO PHENOL HCl), 4-Amino-1-hydroxy-2-methoxymethylbenzene (2-METHOXYMETHYL-p-AMINOPHENOL HC1), 1,3-Bis(N(2-Hydroxyethyl)N(4-amino-phenyl)amino)-2-propanol (HYDROXYPROPYL-BIS-(N-HYDROXY-ETHYL-p-PHENYLENEDIAMINE)HCL), 6-Hydorxyindole (6-HYDROXY-INDOLE), 2,3-Indolinedione (ISATIN), , 1-Phenyl-3-methyl-5-pyrazolone-2,4-dihydro-5,2-phenyl-3H-pyrazole,3-one, 2-Amino-3-hydroxypyridine (2-AMINO-3-HYDROXYPYRIDINE), 5-Amino-salicylic acid, 1 -Methyl-2,6-bis(2-hydroxyethylamino)benzene (2,6-HYDROXYETHYLAMINO-TOLUENE), 4-Hydroxy-2,5,6-triaminopyrimidine (2,5,6-TRIAMINO-4-PYRIMINOL SULPHATE), 2,2'-[1,2-Ethanediyl-bis-(oxy-2,1-ethanediyloxy)]-bis-benzene-1,4-diamine (PEG-3,2',2'-DI-p-PHENYLENEDIAMINE), 5,6-Dihydroxyindoline (DIHYDROXYINDOLINE), N,N-Dimethyl-3-ureidoaniline (m-DIMETHYL-AMINO-PHENYLUREA), 2,4-Diamino-5-fluortoluenesulfatehydrate (4-FLUORO-6-METHYL-m-PHENYLENEDIAMINE SULPHATE) and 1-Acetoxy-2-methylnaphthalene (1-HYDROXYYETHYL-4,5-DIAMINOPYRAZOLE SULPHATE). These can be used in the molecular form or in the form of peroxide-compatible salts.

The hair colouring compositions of the present invention may also include non oxidative hair dyes. i.e. direct dyes which may be used alone or in combination with the above described oxidative dyes. Suitable direct dyes include azo or anthraquinone dyes and nitro derivatives of the benzene series and or melanin precursors and mixtures thereof. Such direct dyes are particularly useful to deliver shade modification or highlights. Particularly preferred are Basic Red 51, Basic Orange 31, Basic Yellow 87 and mixtures thereof.

The hair dye compositions of the present invention will generally comprise from about 0.001% to about 10% of dyes. For example compositions providing 1ow intensity dyeing such as natural blonde to light brown hair shades generally comprise from about 0.001% to about 5%, preferably from about 0.1 % to about 2%, more preferably from about 0.2% to about 1% by weight of dyeing composition of precursors and couplers. Darker shades such as browns and black typically comprise from 0.001% to about 10% by weight, preferably from about 0.05% to about 7% by weight, more preferably from about 1% to about 5% of precursors and couplers.

### Polymers

The composition of the present invention may optionally further comprise at least about 0.01 % of polymer as an additional thickener, rheology modifier, stabilizer and/or conditioning agent as described below.

The polymer can be chosen, for example, from associative polymers. As used herein, the expression "associative polymer" means an amphiphilic polymer comprising both hydrophilic units and hydrophobic units, for example, at least one C8-C30 fatty chain and at least one hydrophilic unit. Representative associative polymers that may be used are associative polymers chosen from:
(i) nonionic,amphiphilic polymers comprising at least one fatty chain and at least one hydrophilic unit; for example celluloses or hydroxyethylcelluloses modified with groups comprising at least one fatty chain, hydroxypropyl guars modified with groups comprising at least one fatty chain, polyether urethanes comprising at least one fatty chain, copolymers of vinylpyrrolidone and of fatty-chain hydrophobic monomers, copolymers of hydrophilic acrylates or methacrylates and of hydrophobic monomers comprising at least one fatty chain.
(ii) anionic amphiphilic polymers comprising at least one hydrophilic unit and at least one fatty-chain unit; these, for example, may be chosen from those comprising at least one fatty-chain allyl ether unit and at least one hydrophilic unit comprising an ethylenic unsaturated anionic monomeric unit, or from those comprising at least one hydrophilic unit of unsaturated olefinic carboxylic acid type, and at least one hydrophobic unit of the type such as a (C8-C30) alkyl ester or oxylakylenated (C8-C30) alkyl ester of an unsaturated carboxylic acid; anionic amphopilic polymers may be further cross-linked.
(iii) cationic amphiphilic polymers comprising at least one hydrophilic unit and at least one fatty-chain unit; these, for example, may be chosen from quaternized cellulose derivatives and polyacrylates comprising amino side groups.
(iv) amphoteric amphiphilic polymers comprising at least one hydrophilic unit and at least one fatty-chain unit; mention may be made, for example, of methacrylamidopropyltrimethylammonium chloride/acrylic acid/C10-C30 alkyl methacrylate copolymers, wherein the alkyl radical is, for example, a stearyl radical.
   Further, the polymer can be chosen from crosslinked acrylic acid homopolymers, crosslinked copolymers of (meth)acrylic acid and of (C1-C6)alkyl acrylate or polysaccharides. The polymer may also serve as conditioning agents, as described below.

Particularly preferred are acrylates/stereath-20 methacrylate copolymer (Aculyn 22 supplied by Rohm and Haas) and acrylates copolymer (Aculyn 33 supplied by Rohm and Haas) and Xanthan gum, Carbopol, Rheozan and mixtures thereof. The polymer will generally be used at levels of from about 0.01% to about 20.0% by weight of the composition, preferably of from about 0.1 % to about 5%.

### Conditioning agent

The compositions of the present invention may comprise or are used in combination with a composition comprising a conditioning agent. Conditioning agents suitable for use herein are selected from silicone materials, amino silicones, fatty alcohols, polymeric resins, polyol carboxylic acid esters, cationic polymers, cationic surfactants, insoluble oils and oil derived materials and mixtures thereof. Additional materials include mineral oils and other oils such as glycerin and sorbitol.

The conditioning agent will generally be used at levels of from about 0.05% to about 20% by weight of the composition, preferably of from about 0.1% to about 15%, more preferably of from about 0.2% to about 10%, even more preferably of from about 0.2% to about 2%.

Particularly useful conditioning materials are cationic polymers and silicones. Conditioners of cationic polymer type may be chosen from those already known by those skilled in the art as improving at least one cosmetic properties of keratin fibres treated with a cosmetic composition. Cationic polymers can be chosen from those comprising units of at least one amine group chosen from primary, secondary, tertiary and quaternary amine groups that may either form part of the main polymer chain, or be borne by a side substituent that is directly attached to the main polymer chain.

Silicones can be selected from polyalkylsilioxane oils, linear polydimethylsiloxane oils containing trimethylsilyl or hydroxydimethylsiloxane endgroups, polymethylphenylsiloxane polydimethylphenylsiloxane or polydimethyldiphenylsiloxane oils, silicone resins, organofunctional siloxanes having in their general structure one or a number of organofunctional group(s), the same or different, attached directly to the siloxane chain. Said organofunctional group(s) are selected from: polyethyleneoxy and /or polypropyleneoxy groups, (per)fluorinated groups, thiol groups, substituted or unsubstituted amino groups, carboxylate groups, hydroxylated groups, alkoxylated groups, quaternium ammonium groups, amphoteric and betaine groups. The silicone can either be used as a neat fluid or in the form of an pre-formed emulsion. Particularly preferred are amino silicones.

### Chelants

According to the present invention the compositions may comprise chelants. Chelants are well known in the art and refer to a molecule or a mixture of different molecules each capable of forming a chelate with a metal ion. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference.

Examples of chelants suitable for use herein include EDDS (ethylenediaminedisuccinic acid), carboxylic acids (in particular aminocarboxylic acids), phosphonic acids (in particular aminophosphonic acids) and polyphosphoric acids (in particular linear polyphosphoric acids), their salts and derivatives.

Chelants may be incorporated into the composition of the present invention as stabilizers and or preservatives. In addition it has also been found that chelants provide hair fibre damage benefits and thus they may be utilized in order to further improve the hair damage profile of the present invention. Levels of chelants in the present invention may be as low as about 0.1%, preferably at least about about 0.25%, more preferably about 0.5% for the most effective chelants such as diamine-N,N'-dipolyacid and monoamine monoamide-N,N'-dipolyacid chelants (for example EDDS). Less effective chelants will be more preferably used at levels of at least about 1%, even more preferably above about 2% by weight of the composition, depending of the efficiency of the chelant. Levels as high as about 10% can be used, but above this level significant formulation issues may arise.

### Solvents

The compositions of the present invention optionally comprise a solvent system, in addition to water. As used herein, the term solvent system refers to a solvent system comprising all the solvents in the composition with the exception of water.

Suitable solvents for use in the solvent system herein include, but are not limited to, amides, esters, ethers, ketones, cyclic amides, cyclic esters, cyclic ketones, cyclic ethers, and mixtures thereof. Nonlimiting examples of such solvents include ethyl formate, dimethyl isosorbide, acetylacetone, 2-butanone, acetone, methyl acetate, ethyl acetate, propyl acetate, ethoxyethanol, dipropylene glycol monomethyl ether, butyl lactate, t-butyl alcohol, phenyl acetate, 2-propoxyethanol, isopropoxyethanol, methoxypropanol, isopropyl lactate, hexyl alcohol, butoxyethanol, tripropylene glycol (PPG-3), triacetin, methoxyethanol, isopropyl alcohol, PEG-8, methyl lactate, PEG-6, PEG-5, PEG-4, N-methylpyrrolidone, propyl alcohol, dipropylene glycol (PPG-2), acetonitrile, phenoxyethanol, triethylene glycol, hexylene glycol, ethyl alcohol, γ-butyrolactone, butylene glycol, propylene carbonate, dimethyl sulfoxide, diethylene glycol, ethoxydiglycol, propylene glycol, pyrrolidone, pyrrolidone-2, methyl alcohol, ethylene carbonate, ethylene glycol, acetamide, glycerin, butyl carbitol, 1,3-dioxolane, dimethoxymethane, 1,2-hexanediol, dipropylene glycol butyl ether, dipropylene glycol t-butyl ether, propionaldehyde, diethoxymethane and glycerol formal.

Preferred solvents for use in the solvent system herein include lower alkanols, C2-C6 polyols, glycol mono-lower alkyl ethers, diglycol mono-lower-alkyl ethers, and N-lower alkylpyrrolidones. The term "lower" refers to the number of carbon atoms being 3 or less. Specific examples include lower alcanols such as Ethanol, Isopropyl alcohol, lower polyols such as ethylene glycol, propyleneglycol, 1,3-butanediol, diethyleneglycol, glycerine; glycol monoethers such as 2-methoxyethanol and 2-ethoxyethanol; diglycol mono-lower alkyl ethers such as methoxydiglycol, ethoxydiglycol and N-lower-alkylpyrrolidones such as N-methylpyrrolidone and N-ethylpyrrolidone.

### Radical Scavenger

The hair colouring and bleaching composition of the present invention may comprise a source of radical scavenger which may be used as an electrolyte for the worm-like micelle phase thickening system of the present invention. As used herein the term radical scavenger refers to a species that can react with a radical, preferably carbonate radical to convert the radical by a series of fast reactions to a less reactive species.

Suitable radical scavengers for use herein include compounds according to the general formula:

(I): R¹-Y-C(H)(R³)-R⁴-(C(H)(R⁵)-Y-R⁶)ₙ

wherein Y is NR², O, or S, preferably NR², n is 0 to 2, and wherein R⁴ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R³ or R⁵ to create a 5, 6 or 7 membered ring; and wherein R¹, R², R³, R⁵, and R⁶ are monovalent and are selected independently from: (a), (b) and (c) described herein above, or H.

Preferably, R⁴ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R⁴ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the R⁴ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S , and N; more preferred are O, and N; and most preferred is O.

Preferably, R¹, R², R³, R⁵, and R⁶ are selected independently from any of the systems defined for R⁴ above, and H. In alternative embodiments, any of R¹, R², R³, R⁴, R⁵, and R⁶ goups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group of N-linked monovalent substituents consisting of NA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COO¹, CON₃, CONA¹₂, CONA¹COA², C(=NA¹)NA¹A², CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.

For the groups (b) to (e), described above, A¹, A², and A³ are monovalent and are independently selected from: (1) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and 1.

Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (II): 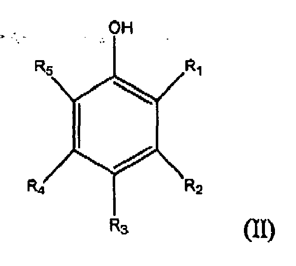 wherein R₁, R₂, R₃, R₄, and R₅ are each independently selected from H,COOM⁺, Cl, Br, SO₃⁻M⁺, NO₂, OCH₃, OH or a C¹ to C¹⁰ primary or secondary alkyl and M is either H or alkali metal. Preferably, the above-described radical scavengers have a pKa of more than 8.5 to ensure protonation of the hydroxy goup.

Other suitable radical scavengers for use herein include hose selected from group (III) benzylamine, imidazole, di-tert-butylhydroxytoluene, hydroquinone, guanine, pyrazine, piperidine, morpholine, methylmorpholine, 2methyoxyethylamine, and mixtures thereof.

Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol,5-anlino-1-pentanol, 1-amino-2-propanol, 1-amino-2-butanol, 1-amino-2-pentanol, 1-amino-3-pentanol, l-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1 % to about 10% by weight, preferably from about 1% to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

### Surfactants

According to the present invention the hair colouring or bleaching compositions may comprise in addition to the amide surfactants described herein above additional surfactants. Suitable surfactant may be selected form anionic, non-ionic, cationic amphoteric, zwitterionic and mixtures thereof.

Preferably, the anionic surfactants are selected from alkyl sulphates, alkyl phosphates, alkyl ether phosphates, alkyl ether sulphates, alkyl glyceryl sulphonates, alkyl Glyceryl ether sulphonates, N-acyl sarcosinates, N-acyl taurates, acyl lactylates and carboxyalkyl ether of alkyl polyglucosides and fatty acid salts and alkyl ether carboxylates and mixtures thereof Yet more preferably, the anionic surfactants are selected from N-acyl sarcosinates, alkyl sulphates, alkyl phosphates, alkyl ether sulphates, alkyl ether phosphates having in average 1 to 20, preferably 1-10 and most preferably 1-3 ethylene oxide units. The anionic surfactants may also be preferably selected from those having an alkyl or acyl radical comprising 16 carbon atoms with on average 1.6 methyl branches per molecule or at least one unsaturated carbon bond.

Preferably the cationic surfactants are selected from quaternary ammonium salts or amido-amines having at least one fatty chain comprising from least about 2 to 30 carbon atoms and mixture thereof. Preferably, the cationic surfactants are selected from those having an alkyl or acyl radical comprising 8 to 18 carbon atoms and a Kraft point of less than 25°C.

The amphoteric or zwitterionic surfactants can be selected, for example, from aliphatic secondary and tertiary amine derivatives in which the aliphatic radical is chosen from linear and branched chains comprising from 8 to 22 carbon atoms and comprising at least one water-soluble anionic group (for example carboxylate, sulphonate, sulphate, phosphate or phosphonate); mention may also be made of (C₈-C₂₀)alkylbetaines, sulphobetaines; (C₈-C₂₀)alkylamido(C₁-C₆)alkylbetaines, (C₈-C₂₀)alkylamido(C₁-C₆)alkylsulphobetaines and mixtures thereof.

Among the amine derivatives, mention may be made of the products sold under the name Miranol, as described, for example, in U.S. Pat. Nos. 2,528,378 and 2,781,354 and having the structures of:

R₂-CON HCH₂CH₂―N⁺(R₃)(R₄)(CH₂COO⁻) (VI)

in which: R₂ is chosen from alkyl radicals derived from an acid R₂-COOH present in hydrolysed coconut oil, and heptyl, nonyl and undecyl radicals, R₃ is a β-hydroxyethyl group and R₄ is a carboxymethyl group; and of

R₅-CONHCH₂CH₂―N(B)(C) (VII)

wherein B represents -CH₂CH₂OX', C represents -(CH₂)₂-Y', with z=1 or 2, X' is chosen from the -CH₂CH₂-COOH group and a hydrogen atom, Y' is chosen from-COOH and -CH₂-CHOH-SO₃H radicals, R₅ is chosen from alkyl radicals of an acid R₅-COOH present in coconut oil or in hydrolysed linseed oil, alkyl radicals, such as C₇, C₉, C₁₁ and C₁₃ alkyl radicals, a C₁₇ alkyl radical and its iso form, and unsaturated C₁₇ radical. These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid. Salts of diethyl aminopropyl cocoaspartamid can be also used.

The nonionic surfactants are compounds that are well known (see, for example, in this respect "Handbook of Surfactants" by M. R. Porter, published by Blackie & Son (Glasgow and London), 1991, pp. 116-178). They can be selected, for example, from polyethoxylated, polypropoxylated and polyglycerolated fatty acids, alkyl phenols, a-diols and alcohols preferably comprising a fatty chain comprising, for example, from 8 to 18 carbon atoms, it being possible for the number of ethylene oxide or propylene oxide groups to range, for example, from 2 to 200 and for the number of glycerol groups to range, for example, from 2 to 30. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide and their momoethanolamine and diethanolamine derivatives, polyglycerolated fatty amides, for example, comprising on average from 1 to 5, and such as from 1.5 to 4, glycerol groups; polyethoxylated fatty amines such as those containing from 2 to 30 mol of ethylene oxide; oxyethylenated fatty acid esters of sorbitan preferably having from 2 to 30 mol of ethylene oxide; fatty acid esters of sucrose, fatty acid esters of polyethylene glycol, alkylpolyglycosides, N-alkylglucamine derivatives, and amine oxides such as (C₁₀-C₁₄)alkylamine oxides or N-acylaminopropylmorpholine oxides and mixtures thereof. Preferably the nonionic surfactants are selected from polyethoxylated fatty alcohols, more preferably polyethoxylated fatty alcohols having an alkyl chain length of from 8 to 20 carbon atoms and a degree of ethoxylation of between 2 and 20 ethylene oxide units.

### Method of Use

It is understood that the examples of methods of use and embodiments described herein are for illustrative purposes only and that various modifications or changes in light thereof will be suggested to one skilled in the art without departing from the scope of the present invention.

The resultant mixed hair colouring or bleaching compositions according to the present invention thus preferably have a viscosity of from 1000 to 60000 cPs, preferably from 2000 to 30000 cPs and most preferably from 3000 to 25000 cPs. Moreover, prior to mixing the hair dye component (second component) and or the oxidizing component (first component) may have viscosity of less than 1000 cPs; such compositions are often referred as "thin-thin" or "liquid" colorant. The viscosity of the resultant mixture of first oxidative and second components i) and ii) in other words the hair colouring or bleaching composition that is applied on head is from 1000 to 60000 cPs, preferably from 2000 to 30000, more preferably form 3000 to 25000 cPs.

In another embodiment of the present invention the oxidative hair dye or bleaching compositions may comprise as an optional fourth component a colour referesher composition. Such colour refresher compositions comprise at least one pre-formed dye and may be applied to the hair immediately after the oxidative colour i.e. from about 1 minute after oxidative hair dye or bleach application to 60 days after the application. These colour refresher composition can be used to increase the intial colour obtained and or boost the colour during the wash and style cycle until the next oxidative colouring or bleaching event.

The present invention may be utilized in a variety of packaging and dispensing devices. These dispensing devices can come in the form of separate devices which may be used independently or in combination with one another. Typically, the hair colouring or bleaching compositions are contained within separate single or multi compartment containers so that the compositions can be stored separately from one another before use. The compositions are then mixed together by a mixing means and then applied to the consumer's hair by an application means.

The most common packaging device which can be used for the present invention involves storing the developer in a container such as a bottle, tube, aerosol, or a sachet and separately storing the dye lotion in an additional compartment within the developer container or in a separate container which may be identical such as a dual sachet or aesrosol systems for example or different such as a bottle and tube system.

The consumer may mix the developer lotion and the dye lotion by any means. This may simply involve the use of a mixing bowl into which the lotions are dispensed and then mixed, preferably using a mixing means such as a tool. Alternatively it may involve the addition of one of the lotions into the container of the other lotion, (typically the dye lotion is added to the developer lotion), followed by manual shaking or mixing with a tool. Another system involves the perforation or displacement, of a seat located between the separate compartments of the dye and developer lotion within a single container or sachet followed by manual mixing within the container or in a separate and or additional container.

An example of such devices are the so called 'twist and go' devices. These devices allow the consumer to twist the base of a container holding the dye which enables a communication port to open that exposes the base of the bottle holding the dye and the top of the bottle holding the developer. The two components are mixed and the consumer dispenses the product by squeezing the flexible top portion of the bottle for dispensing.

Alternatively more complex devices may be utilised, whereby the lotions are mixed upon actuation of dispensing. An example of such a complex system is a dual aerosol system e.g. bag-in-can or piston. The dye and developer are stored separately in two aerosol cans within one device, a propellant being used to pressurize the contents of the can or bag in can or piston and a valve providing the control of dispensing. When the consumer actuates the valve, the dye and developer are dispensed simultaneously out of the cans and are mixed together via a static mixer just before dispensing the product onto the hair. The ratio of the dye and developer can be manipulated by the viscosity of the products, the can pressure, or by altering the flow channel sizes through the valve. Additionally, the product can be foamed and delivered via a mousse form.

Another example of such a complex system utilises a dual piston screw system. The dye and the developer are kept in separate piston cylinder systems within the system and when the consumer actuates a button, two screws are rotated such that the dual pistons inside pressurize the liquid in the cylinders and thus force the products to move through a mixing station and out of the nozzle for dispensing. The ratios of the dye and the developer can be manipulated by the diameter of the cylinder of the package. Additionally, an in line static mixer can be used to aid mixing and such a system can be completely disposable or completely refillable.

Yet another system utilises one or more manually actuated pumps. The product may be premixed in a collapsible sachet. When the consumer actuates the pump, the liquid inside the pump is dispensed. As the manually actuated pump returns to the upright position it forces product from a collapsible sachet. Alternatively, a dual system can be installed whereby two sachets and two pumps ,are used to deliver the dye and the developer lotions to the hair. Alternatively, a single pump connected to two sachets can deliver the product by incorporating the mixing point within the pump. Another embodiment uses a rigid bottle and a dip tube to connect the product to the pump system. Finally, a delaminating bottle can be used in combination with a manually actuated pump where the inner layer of the bottle separates from the outer layer of the bottle which forces the contents of the bottle to be emptied.

Typically these complex systems offer the advantage of product application independently of the orientation of the product.

The devices described herein above can also be used in combination with a product delivery and or application tool to aid application of the product onto the hair. Again these devices may be of a very simple nature such as a nozzle attached to one of the containers or a separate applicator device such as a comb or brush. Such combs and brushes can be adapted in order to achieve particular effects, whether it be quick and even coverage or root/hairline touch up, or highlights or streaks. Alternatively, the container or one of the containers may be provided with a comb attached to or instead of the dispensing nozzle whereby the product is dispensed through hollow tines and dispensing apertures located in the comb tines. The comb tines may be provided with single or multiple openings along the tines to improve product application and evenness especially root to tip. Product dispensation can be achieved by mechanical pressure applied to the container for example delaminating bottles or any of the mechanisms described hereinabove. The comb may be provided on the container such as to facilitate easy application and may be positioned vertically (so called verticomb) or at an angle to allow the consumer to access all areas. All devices may be designed to have inter-changeability, so that a range of different tools for hair application can be provided to the consumer.

The application devices may also include devices which assist in achieving particular effects such as highlighting, such as highlighting combs, brushes and tools, foils and highlighting caps.

Additional device technology can be used to assist in the penetration of the product into the hair. Examples of such technology include heating devices, ultraviolet light devices and ultrasound devices.

### Test Methods

### Examples

The following examples illustrate oxidative dye compositions according to the present invention. It is understood that the examples and embodiments described herein are for illustrative purposes only and that various modifications or changes in light thereof will be suggested to one skilled in the art without departing from the scope of the present invention.

### Examples 1-13 (mixed compositions)

| **Ingredient** | **1** | **2** | **3** | **4** | **5** |
|---|---|---|---|---|---|
| Ammonium Carbonate | 2 | 7.5 | 4 | 5 | 5 |
| Ammonium Hydrogen Carbonate | - | - | - | - | - |
| Ammonium Carbamate | - | - | - | - | - |
| Sodium Glycinate | 1.0 | 5.0 | 2.0 | 2.5 | 2.5 |
| Lauryl Methylglucamide (*HATCO HXL7658*) | | 5 | | 10 | |
| Ethoxy-5-Behenamide | 5 | | 5 | | |
| Ethoxy-10-Erucamide | 15 | | 15 | | 15 |
| Oleth 5 | | 2.5 | | 5 | |
| Oleth 2 | | 2.5 | | 5 | |
| Cetyltrimethyl Ammonium Chloride | - | - | - | - | - |
| Sodium Myristoyl Sarcosinate | - | - | 0.1 | - | - |
| Cocamidopropyl Betaine | - | - | - | 0.1 | - |
| p-phenylene diamine | 0.1 | | 0.8 | - | 0.1 |
| p-amino phenol | 0.4 | 0.3 | - | 0.3 | 0.4 |
| 2,5-diamino-toluene sulphate | - | 0.1 | - | 0.1 | - |
| m-aminophenol | - | - | 0.2 | - | - |
| Resorcinol | 0.4 | 0.5 | - | 0.5 | 0.4 |
| napthol | - | - | 0.03 | - | - |
| 4-amino-2-hydroxy toluene | 0.3 | 0.2 | - | 0.2 | 0.3 |
| Phenyl methyl pyrazalone | - | - | - | 0.2 | - |
| 1-hydroxyethyl-4,5-diamino pyrazole sulphate | - | - | - | 0.3 | - |
| Basic red 51 | - | 0.1 | - | 0.1 | - |
| Basic yellow 87 | - | 0.2 | - | 0.2 | - |
| Hydrogen Peroxide (35% active) | 2.85 | 11.0 | 11.0 | 17.14 | 12.85 |
| Amidomethicone(DCAP 6087) | - | - | - | 1.0 | - |
| Xanthan gum | - | 0.2 | - | - | - |
| EDTA (tetra-sodium salt) | 0.1 | 0.1 | 0.1 | 0.1 | 0.1 |
| Sodium sulphite | 0.1 | 0.1 | 0.1 | 0.1 | 0.1 |
| Ascorbic Acid | 0.1 | 0.1 | 0.1 | 0.1 | 0.1 |
| Propylene Glycol | 3 | 3 | 3 | 3 | 3.0 |
| Etidronic Acid | 0.1 | 0.1 | 0.1 | 0.1 | 0.1 |
| PEG150/ Stearyl Alcohol/ SMDI copolymer | - | - | - | - | 1.5 |
| pH adjust to pH 9.0 | qs | qs | qs | qs | qs |
| Water | qs | qs | qs | qs | qs |

| **Ingredient** | **6** | **7** | **8** | **9** | **10** |
|---|---|---|---|---|---|
| Ammonium Carbonate | 5 | 10 | - | 5 | 5 |
| Ammonium Hydrogen Carbonate | - | - | 4.0 | | |
| Ammonium Carbamate | - | - | 4.0 | - | - |
| Sodium Glycinate | 2.5 | 10 | 2.5 | - | 2.5 |
| Lauryl Methylglucamide (*HATCO HXL 7658*) | | 5 | | 10 | |
| Ethoxy-5-Behenamide | 5 | | 5 | | 10 |
| Ethoxy-10-Erucamide | 15 | | 15 | | 30 |
| Oleth 5 | | 2.5 | | 5 | |
| Oleth 2 | | 2.5 | | 5 | |
| Cetyltrimethyl Ammonium Chloride | - | - | - | - | 2.0 |
| p-phenylene diamine | 0.8 | - | 0.6 | 0.1 | 0.8 |
| p-amino phenol | - | - | - | 0.4 | - |
| 2,5-diamino-toluene sulphate | - | - | 0-2 | - | - |
| m-aminophenol | 0.2 | - | 0.1 | - | 0.2 |
| Resorcinol | - | - | - | 0.4 | - |
| napthol | 0.03 | - | 0.2 | - | 0.03 |
| 4-amino-2-hydroxy toluene | - | - | - | 0.3 | - |
| Phenyl methyl pyrazalone | - | - | - | - | - |
| 1-hydroxyethyl-4,5-diamino pyrazole sulphate | - | - | - | - | - |
| Basic red 51 | - | - | 0.2 | - | - |
| Basic yellow 87 | - | - | 0.3 | - | - |
| Hydrogen Peroxide (35% active) | 8.6 | 12.85 | 11.0 | 12.85 | 17.14 |
| Amidomethicone (DCAP 6087) | - | - | - | - | - |
| Polyquatcmium-22 (Merquat 295) | 0.1 | - | - | 0.1 | - |
| Polyquatemium-37 & Mineral oil (Salcare SC95) | 0.2 | - | - | 0,2 | - |
| Xanthan gum | - | - | - | - | 0.2 |
| EDTA (tetra-sodium salt) | 0.1 | 0.1 | 0.1 | 0.1 | 0.1 |
| Sodium sulphite | 0.1 | 0,1 | 0.1 | 0.1 | 0.1 |
| Ascorbic Acid | 0.1 | 0.1 | 0.1 | 0.1 | 0.1 |
| Propylene Glycol | 3.0 | 3.0 | 3.0 | 3.0 | 3.0 |
| Etidronic Acid | 0.1 | 0.1 | 0.1 | 0.1 | 0.1 |
| pH adjust to pH 9.0 | qs | qs | qs | qs | qs |
| Water | qs | qs | qs | qs | qs |

| **Ingredient** | **11** | **12** | **13** |
|---|---|---|---|
| Ammonium Hydroxide (29% solution) | 4.5 | - | 4.5 |
| Ammonium Carbonate | - | 5.0 | - |
| Sodium Glycinate | - | 2.5 | - |
| Lauryl Methylglucamide (*HATCO HXL 7658*) | | 5 | |
| Ethoxy-5-Behenamide | 5 | | 5 |
| Ethoxy-10-Erucamide | 15 | | 15 |
| Oleth 5 | | 2.5 | |
| Oleth 2 | | 2.5 | |
| Ceteareth-25 | - | 1.5 | - |
| Cetyl Alcohol | - | 1.12 | - |
| Stearyl Alcohol | - | 1.12 | - |
| p-phenylene diamines | 0.1 | 0.1 | 0.1 |
| p-amino phenol | 0.4 | 0.4 | 0.4 |
| Resorcinol | 0.4 | 0.4 | 0.4 |
| 4-amino-2-hydroxy toluene | 0.3 | 0.3 | 0.3 |
| Hydrogen Peroxide (35% active) | 2.85 | 17.14 | 11 |
| Polyquatemium-22 (Merquat 295) | - | - | 0.1 |
| Polyquatemium-37 & Mineral oil (Salcare SC95) | - | - | 0.2 |
| Xanthan gum | - | 0.5 | - |
| Aculyn 33 | - | - | - |
| EDTA (tetrasodium salt) | 0.1 | 0.1 | 0.1 |
| Disodium EDDS | - | - | - |
| Sodium Citrate | - | - | - |
| Sodium sulphite | 0.1 | 0.1 | 0.1 |
| Ascorbic Acid | 0.1 | 3.0 | 0.1 |
| Propylene Glycol | 3.0 | 3.0 | 3.0 |
| Etidronic Acid | 0.1 | 0.1 | 0.1 |
| pH | 10 | 9.0 | 10 |
| Water | qs | qs | qs |

For examples 1-13, using the method to defined hereinabove the viscosity of the mixed systems are 6000 to 10000 cPs.

### Examples 14-17

The following hair colouring compositions are prepared (Part A);

| | **Ingredient** | **14** | **15** |
|---|---|---|---|
| 1 | Ammonium Carbonate | 10 | 15 |
| 2 | Lauryl Methylglucamide (*HATCO HXL 7658*) | | 5 |
| 3 | Ethoxy-5-Behenamide | 5 | |
| 4 | Ethoxy-10-Erucamide | 15 | |
| 5 | Oleth 5 | | 2.5 |
| 6 | Oleth 2 | | 2.5 |
| 7 | Xanthan | - | - |
| 8 | Para-phenylene-diamine | 0.6 | - |
| 9 | Para-aminophenol | - | 0.30 |
| 10 | Meta-aminophenol | 0.2 | - |
| 11 | Resorcinol | - | - |
| 12 | Naphthol | 0.03 | - |
| 13 | Phenyl methyl pyrazalone | 0.2 | - |
| 14 | 1-hydroxyethyl-4-5-diamineo pyrazole | 0.3 | - |
| 15 | 2,5 diaminotoluene sulphate | - | - |
| 16 | 4 amino-2-hydroxytoluene | - | - |
| 17 | EDTA(tetrasodiumsalt) | 0.1 | 0.1 |
| 18 | Sodium sulphite | 0.1 | 0.1 |
| 19 | Ascorbic Acid | 0.1 | 0.1 |
| 20 | Sodium Glycinate | 5 | 15 |
| 21 | Propylene Glycol | 4.00 | 4.00 |
| 22 | IPA | 4.00 | 4.00 |
| 23 | pH adjust to pH 9.0 | qs | qs |
| 24 | Water | qs | qs |

The viscosity of the composition of Example 15 and 16 (part A) is below 1000 cPs i.e. it is a thin-thin composition.

The following developer compositions are prepared (Part B):

| | **Ingredient** | **Formulation #** | |
|---|---|---|---|
| | | **16** | **17** |
| 1 | Hydrogen peroxide (35%) | 17.0 | 25.71 |
| 2 | Etidronic Acid | 0.2 | 0.2 |
| 3 | Polyquatemium-22 (Merquat 295) | 0.1 | - |
| 4 | Polyquatemium-37 & Mineral oil (Salcare SC95) | 0.2 | - |
| 5 | Aculyn 22 | 2.5 | 2.5 |
| 6 | Structure 3001 | 2.5 | 2.5 |
| 7 | Water | qs | qs |

Part A and Part B are mixed prior to application on hair and the viscosity of the mixed formulations is within the range of 1000 to 60000 cPs.

### Viscosity Test method

The viscosity is measured using Brookfield viscometers with cone and plate attachment. For viscosities in the range of 0-12000 cPs the Brookfield DV-11 viscometer with S42 plate is used. 2ml sample of the composition is equilibrated at 26.7°C for three minutes before the readings are taken at 1 rpm. For viscosities in the range of 12,000-50,000 cPs the Brookfield DV-1 viscometer with S52 plate is used. 0.5ml sample of the composition is equilibrated for 1 minute at 26.7°C before the readings are taken at 1 rpm.

## Claims

1. A hair colouring or bleaching composition comprising
i) At least 0.25 mole/l of a source of carbonate, carbamate, hydrogencarbonate or peroxymonocarbonate ions and mixtures thereof,
ii) at least one oxidizing agent and
iii) at least one amide surfactant selected from polyoxyethylene amides or polyhydroxy amides or mixtures thereof.

2. A hair colouring and or bleaching composition according to claim 1, wherein said amide surfactant is a polyoxyethylene amide according to the formula R-(OCH₂CH₂)ₓ-(OCH₂)_{y}-C(O)NH(CH₂CH₂O)₂-H, wherein x is independently selected from 0 to 100, y is 0 or 1, z is independently selected from 1 to 100, and R is independently selected from alkyl, alkenyl or alkylaryl groups having from 8 to 30 carbon atoms.

3. A hair colouring and or bleaching composition according to claim 1, wherein said amide surfactant is a polyhydroxy amide according to the formula RC(O)N(R')CH₂-(CHOH)ₙ-H, wherein n is independently selected from 4 to 6, R' is hydrogen or methyl group, R is independently selected from alkyl, alkenyl or alkylaryl groups having from 8 to 30 carbon atoms.

4. A hair colouring and or bleaching composition according to claim 1, wherein said Composition comprises
i) from 1% to 35% by weight, preferably from 5% to 20% of said source of carbonate, carbamamte, hydrogencarbonate or peroxymonocarbonate ions,
ii) from 1% to 15% by weight, preferably from 3% to 12% of said oxidizing agent
iii) from 1% to 60% by weight, preferably from 5% to 40% of said amide surfactant.

5. A hair colouring and or bleaching composition according to any one of the preceeding claims, wherein said composition further comprises an ionic surfactant selected from alkyl sulphates, alkyl ether sulphates, alkyl phosphates, alkyl ether phosphates, alkyl glyceryl sulphonates, N-acyl sarcosinates, N acyl taurates, acyl lactylates, carboxyalkyl ethers of alkyl polyglucosides, fatty acid salts, alkyl ether carboxylates and mixtures thereof.

6. A hair coloring and or bleaching composition according to any one of the preceding claims, wherein said composition further comprises a cationic surfactant selected from quaternary ammonium salts, amido-amines and mixtures thereof.

7. A hair coloring and or bleaching composition according to any one of the preceeding claims, wherein said composition further comprises a zwitterionic surfactant selected from alkyl-ampho mono- and di-acetates, alkyliminodiacetates, alkylamidopropyl betaines, alkylamido betaines, alkyl betaines, alkyl dimethyl amine oxides, dihydroxyethyl alkyl amine oxides, alkylamine oxides, dihydroxyethylamine oxides and mixtures thereof.

8. A hair coloring and or bleaching composition according to any one of the preceeding claims, wherein said composition further comprises a non-ionic surfactant selected from polyethoxylated, polypropoxylated and polyglycerolated fatty acids; alkyl phenols; α-diols and alcohols; copolymers of ethylene oxide and of propylene oxide; condensates of ethylene oxide and of propylene oxide with fatty alcohols; polyethoxylated fatty amides and their momoethanolamine and diethanolamine derivatives; polyglycerolated fatty amides; polyethoxylated fatty amines, oxyethylenated fatty acid esters of sorbitan; fatty acid esters of sucrose, fatty acid esters of polyethylene glycol; alkylpolyglycosides; N-alkylglucamine derivatives amine oxides and preferably selected from polyethoxylated fatty alcohols and most preferably selected from polyethoxylated fatty alcohols having an alkyl chain length of between 8 and 20 carbon atoms and a degree of ethoxylation between 2 and 20 ethylene oxide units.

9. A hair colouring and or bleaching composition according to claim 1, wherein said composition further comprises at least one radical scavenger.

10. A hair colouring and or bleaching composition according to claim 9, wherein said radical scavenger is selected from potassium, sodium and ammonium salts of glycine, sarcosine, lysine, serine, glutamic acid, and mixtures thereof.

11. A hair colouring and or bleaching composition according to claim 1, wherein said composition has a pH of from 8.4 to 9.5.

12. A hair colouring and or bleaching composition according to any one of the preceding claims, wherein said composition has a viscosity of from 1000cPs to 60000 cPs, preferably from 2000cPs to 30000 cPs and most preferably from 3000cPs to 25000 cPs.

13. A hair colouring and or bleaching composition according to any one of the preceding claims, wherein said composition comprises at least one oxidative dye precursor and or at least one pre-formed dye.

14. A hair colouring and or bleaching composition according to any one of the preceding claims, wherein said composition further comprises at least one polymer, preferably selected from associative polymers, crosslinked acrylic acid homopolymers, associative zwitterionic polymers, crosslinked zwitterionic polymers, crosslinked copolymers of (meth)acrylic acid and of (C1-C6)alkyl acrylate, non-ionic homopolymers and copolymers comprising ethylenically unsaturated monomers of ester and amide type, ammonium acrylate homopolymers and copolymers of ammonium acrylate and of acrylamide or polysaccharides and mixtures thereof.

15. A hair colouring and or bleaching kit comprising
i) an individually packaged first oxidising component comprising at least one oxidizing agent preferably hydrogen peroxide and
ii) an individually packaged second component comprising at least one amide surfactant selected from polyoxyethylene amides or polyhydroxy amides or mixtures thereof and a source of carbonate, carbamate, hydrogencarbonate or peroxymonocarbonate ions.

16. A hair colouring or bleaching kit according to claims 15, wherein the viscosity of said second component ii) is less than 1000 cPs and wherein the viscosity of the resultant composition mixture of said first and second components i) and ii), is from 1000cPs to 60000cPs, more preferably from 2000cPs to 30000 cPs.
